# EUROPEAN PATENT SPECIFICATION

(11) **EP 1 401 534 B1**
(45) Date of publication and mention of the grant of the patent: **03.01.2007**
(21) Application number: 02735907.4
(22) Date of filing: 18.06.2002
(51) Int. Cl.: A61N 1/362, A61N 1/39, A61N 1/05

(54) **DISPOSABLE SHEATH PROVIDING CARDIAC STIMULATION**
WEGWERFHÜLLE FÜR HERZSTIMULATION
GAINE JETABLE PRODUISANT UNE STIMULATION CARDIAQUE

(30) Priority: 22.06.2001 US 887644
(43) Date of publication of application: 31.03.2004
(73) Proprietor: Koninklijke Philips Electronics N.V., 5621 BA Eindhoven (NL)
(72) Inventor: ROCK, Joseph, Internationaal Octrooibureau B.V., NL-5656 AA Eindhoven (NL); FEUERSANGER, Robert, Inter. Octrooibureau B.V., NL-5656 AA Eindhoven (NL)
(74) Representative: van der Veer, Johannis Leendert
(86) International application number: PCT/IB2002/002418
(87) International publication number: WO 2003/000341

(56) References cited:
- US-A- 4 640 298
- US-A- 5 191 885
- US-A- 5 749 833
- US-A- 5 928 270

## Description

Atrial fibrillation (AF) is the most common sustained arrhythmia and is responsible for 365,000 hospital admissions annually according to the American Heart Association, 2001 Heart and Stroke Statistical Update. Dallas, Texas: American Heart Association, 2000. In AF, the two small upper chambers of the heart, the atria, quiver instead of beating effectively. But because of the minimal contribution of atrial contraction to cardiac output, atrial fibrillation is hemodynamically tolerated and not generally regarded as life threatening. Nonetheless, given the ongoing risk of a stroke and the symptoms of dyspnea and fatigue that afflict the patient, attempts are generally made to minimize the time the patient is in AF or to more permanently convert the patient out of AF into a normal sinus rhythm (NSR).

Conversion out of AF to NSR may be spontaneous, or may be induced by pharmacological or electrical means. The electrical treatment for atrial fibrillation is referred to as atrial cardioversion. The present invention safely and effectively converts the patient through atrial cardioversion.

US-A- 5191885 and US-A-5928270 disclose prior art systems of the present invention. In atrial cardioversion, electrical energy is introduced into the body through the use of electrical conductors, typically patches that are placed on the chest. The amount of energy required to convert AF is dependent on both the unique impedance of the patient and the dysrhythmia's response to prior energies.

During these conversions, the patients are at increased risk for thromboembolic events, which is the most significant risk associated with AF and its management. This is because when the blood is not pumped completely out of the atria during normal contractions, the blood may pool and clot. When a normal sinus rhythm is restored as a result of cardioversion, bits of the clot (thrombus) may break off and become emboli in the blood stream. If the embolus becomes lodged in an artery in the brain the patient will suffer an embolic stroke.

In an effort to reduce the risk, two strategies are currently being pursued. The first strategy used by physicians is by treating the patient with prophylactic anticoagulation of three to four weeks of oral warfarin. The second strategy used by physicians is to evaluate the patient for atrial thrombi using a transesophageai echocardiogram (TEE). TEE is a test that allows a cardiologist to view the internal structures of the heart and the heart's major vessel by inserting an ultrasound probe down the throat. The patient is sedated so he or she will be relaxed and unaware of any discomfort during the procedure. Once the TEE probe is inside, the tip of the TEE probe sends out sound waves (ultrasound) that echo within the chest wall cavity. These echoes are picked up and create a picture of the heart that is displayed on a video monitor. If there are no thrombi detected, the patient is started on IV heparin to reduce the risk that the patient might develop a thrombus between the exam and the actual cardioversion perhaps up to 48 hours later. Both strategies seek to reduce the risk of a thromboembolic event by either visually clearing the atria for thrombi or treating them with the expectation of their dissolution. In both cases, an anticoagulation treatment is continued for four weeks postcardioversion.

If the first strategy is used, the patient is left in AF for the period of time that the anticoagulation treatment is performed. According to David I. Silverman, MD and Warren J. Manning, MD, Role of Echocardioqraphv in Patients Undergoing Elective Cardioversion of Atrial Fibrillation. American Heart Association 1998, Circulation 1998; 98:480, it has been shown that a long-term maintenance of sinus rhythm is inversely related to the duration of AF before cardioversion. In addition, the recovery of atrial mechanical function may be inversely related to the duration of AF. Furthermore, there is an increased risk of overall hemorrhagic complications owing to the increased prothrombin times because of the anticoagulation therapy.

Further, if the patient is treated with IV heparin or warfarin, a transesophageai echocardiogram typically would not be performed to determine whether or not blood clots still exist in the atria. It is assumed that after IV heparin or three to four weeks of warfarin, the blood clots have dissolved. This is not always the case and the risk of new thrombi is real.

Thus, the TEE test is gaining acceptance as a way of determining whether there are blood dots within the heart. If the TEE test is negative for thrombi, cardioversion could be performed sooner. This is important because the longer a patient is in AF, the lower the likelihood that once converted out of AF the patient would maintain a normal rhythm. The sooner the patient receives treatment, the better the chances that the treatment stays effective.

A drawback to the TEE approach is that the user must go through two procedures, one for the TEE test itself, which requires a mild sedation, and a second for the actual cardioversion. The TEE approach requires two doses of anesthesia; two visits by the anesthesiologist, two visits to the EP lab and other procedures that are performed more than once, thereby requiring more time and resources and creating extra discomfort for the patient. In addition, current methods for providing cardioversion expose the patient to higher levels of energy, thereby risking myocardial damage. A system is needed to minimize redundant procedures and to increase effectiveness of the cardioversion itself.

In an exemplary embodiment, the present disclosure provides for a system providing cardiac stimulation, including a probe insertable through a mouth into an esophagus of a patient; a disposable sheath slidably covering the probe; a conductor integrated in the sheath; and a transthoracic pad connected to the sheath and providing the cardiac stimulation to the patient in combination with the conductor.

In an exemplary embodiment, the present disclosure provides for a system providing cardiac stimulation, including a first conductor; a second conductor; and a disposable sheath including the first conductor and the second conductor integrated therein, wherein the first and the second conductors are connected to a cardiac resuscitation apparatus via a single cable providing the cardiac stimulation to the patient.

In an exemplary embodiment, the present disclosure provides for a system providing cardiac stimulation, including a probe insertable through a mouth into an esophagus of a patient; a first conductor; a second conductor; and a disposable sheath slidably covering the probe and including the first conductor and the second conductor integrated therein, wherein the first and the second conductors are connected to a cardiac resuscitation apparatus via a single cable providing the cardiac stimulation to the patient.

The present invention is achieved by a system providing cardiac stimulation, including a first group of conductors; a second group of conductors; and a disposable sheath including the first group of conductors and the second group of conductors integrated therein providing a path of least resistance between one of the conductors in the first group of conductors and one of the conductors in the second group of conductors, wherein the first and the second groups of conductors are connected to a cardiac resuscitation apparatus via a single cable to provide the cardiac stimulation to the patient, and further
characterized by the features of claim 1.

The present invention is also achieved by a system providing cardiac stimulation, including a probe insertable through a mouth into an esophagus of a patient; a first group of conductors; a second group of conductors; and a disposable sheath slidably covering the probe and including the first group of conductors and the second group of conductors integrated therein providing a path of least resistance between one of the conductors in the first group of conductors and one of the conductors in the second group of conductors, wherein the first and the second groups of conductors are connected to a cardiac resuscitation apparatus via a single cable to provide the cardiac stimulation to the patient, and further characterized by the features of claim 9.

In an exemplary embodiment, the present disclosure provides for a system providing cardiac stimulation, including a conductor; an inflatable balloon; a disposable sheath including a conductor integrated therein at or near a distal end of the sheath and the inflatable balloon positioned behind the conductor to close a gap between the esophagus and the sheath and push the conductor against a wall of the esophagus to provide the cardiac stimulation to the patient; and a transthoracic electrode pad connected to the sheath and providing the cardiac stimulation to the patient in combination with the conductor.

In an exemplary embodiment, the present disclosure provides for a system having cardiac stimulation, including a probe insertable through a mouth into an esophagus of a patient; a conductor; an inflatable balloon; a disposable sheath slidably covering the probe and including a conductor integrated therein at or near a distal end of the sheath and the inflatable balloon positioned behind the conductor to dose a gap between the esophagus and the sheath and push the conductor into a wall of the esophagus to provide the cardiac stimulation to the patient; and a transthoracic electrode pad connected to the sheath and providing the cardiac stimulation to the patient in combination with the conductor.

In an exemplary embodiment, the present disclosure provides for a method determining whether a defibrillator is connected to a transthoracic pad set or a combination of a transthoracic pad and a probe including a disposable sheath, the method including selecting the pad set or the combination of the pad and the probe including the sheath; detecting the pad set or the combination of the pad and the probe including the sheath; identifying whether the pad set or the combination of the pad and the probe including the sheath is connected to the defibrillator; configuring the defibrillator to use a low-energy setting in response to determined that the combination of the pad and the probe including the sheath is being used; configuring the defibrillator to use a high-energy setting in response to determining that the pad set is being used; and arming and discharging the defibrillator to provide cardiac stimulation to a patient.

Embodiments of the present disclosure not according to the invention are disclosed for more information.

The various objects and advantages of the invention will become apparent and more readily appreciated from the following description of the preferred embodiments, taken in conjunction with the accompanying drawings of which:
Fig. 1 is a diagram illustrating a single conductor sheath with a single transthoracic cardiac stimulation pad, in an exemplary embodiment of the present disclosure
Fig. 2 is a diagram illustrating a dual conductor sheath, in accordance with an exemplary embodiment of the present disclosure,
Fig. 3 is a diagram illustrating multiple conductor sheath, in accordance with an exemplary embodiment of the present invention;
Fig. 4 is a diagram illustrating a single conductor sheath with optional inflatable balloon and associated tubing, in accordance with an exemplary embodiment of the present disclosure
Fig. 5 is a diagram illustrating a sheath with related equipment connected thereto; and
Fig. 6 is a schematic diagram of a process determining whether a cardiac stimulation system is using a conventional transthoracic pad set or a probe including the sheath of the present invention and a single transthoracic pad.

Reference will be now made in detail to the present preferred embodiments of the present disclosure examples of which are illustrated in the accompanying drawings, wherein like reference numerals refer to the like elements throughout. In an exemplary embodiment, the present disclosure provides for a flexible membrane disposable sheath including a conductor embedded or integrated into the sheath, at or near a distal end of the sheath that is acoustically transparent. The sheath slidably covers a probe, such as a transesophageal ultrasound (TEE) probe or an endoscope, to introduce the sheath into the esophagus and perhaps stomach. For illustrative purposes, the present disclosure will be described using the TEE probe. However, an ordinary person skilled in the art will appreciate that an endoscope or any other type of similar device may be used rather than the TEE probe. The sheath is connected to a single transthoracic cardiac stimulation electrode pad. A connector connects the sheath and the transthoracic cardiac stimulation electrode pad to a cardiac resuscitation apparatus, such as a defibrillator. While the sheath is in the esophagus, if a physician determines that cardioversion treatment is necessary to normalize a patient's heartbeat, then the combination of the conductor in the sheath and the transthoracic cardiac stimulation electrode pad may be used to apply the treatment right there and then.

Further, the probe may be placed very close to the right atrium, thereby requiring less energy to convert and lessening the chances of damaging the myocardium with the energy (current) applied during cardioversion. The sheath would include an insulation type coating with suitable dielectric strength inside the sheath cavity to protect transducer elements in the probe from damage by the energy applied during defibrillation, pacing, or cardioversion.

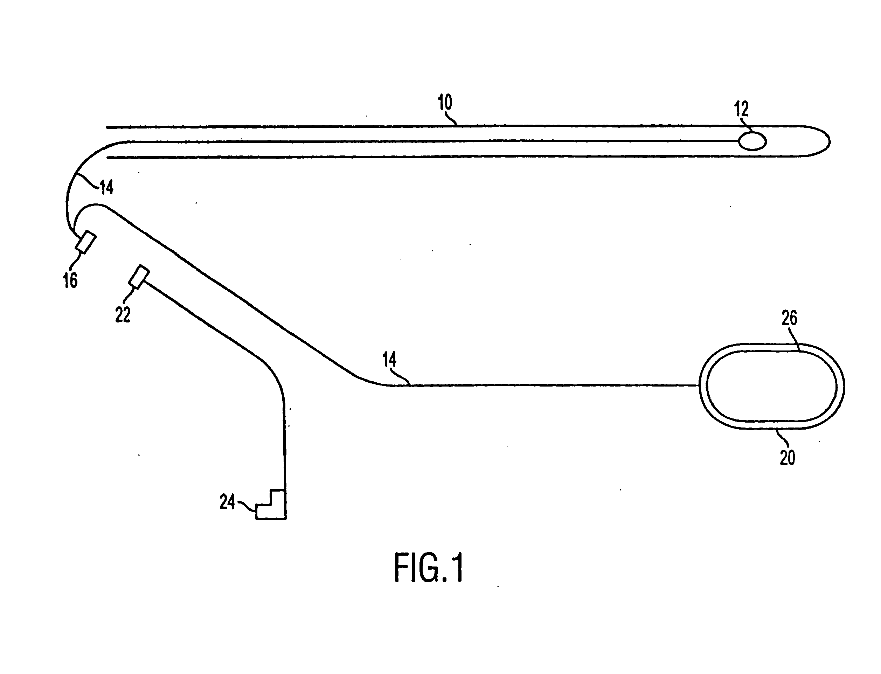

Fig. 1 illustrates a single conductor sheath 10 with a single transthoracic cardiac stimulation electrode pad 20 including a conductor and a hydro-gel or an electro-gel. A conventional elongated, flexible TEE probe (not shown) includes an echo transducer that is positioned at the end of the TEE probe. Once the patient is sedated, the TEE probe is inserted through the patient's mouth into the esophagus. The echo transducer sends out sound waves (ultrasound) that echo within the chest wall cavity. These echoes are picked up and create a picture of the heart that is displayed on a video monitor (not shown). When obtaining an image of the heart, the tip of the TEE probe is placed into the patient's stomach to obtain a gastric view and look back to the heart.

The sheath 10 slidably covers the TEE probe. The sheath 10 is made of an electrical insulative flexible membrane material and is disposable. A cardiac stimulation electrical conductor 12 is integrated in the sheath 10, that is, embedded on the surface skin of the sheath 10 and it is positioned near or at a distal end of the sheath 10. The cardiac stimulation electrical conductor may either partially or completely circumscribe the sheath. An electrically conductive, insulated cable 14 is a flexible stranded cable suitable for carrying sufficient current called for by the total energy in a shock impulse generated by an external defibrillator unit to the cardiac stimulation conductor 12 to provide cardiac stimulation to the patient. Cardiac stimulation includes defibrillation, cardioversion, and pacing. The cable 14 extends from the cardiac stimulation conductor 12 beyond a proximal end of the sheath 10, as illustrated in Fig. 1 to a connector 16. The cable 14 also connects the electrode pad 20 to the connector 16.

In operation, the connector 16 is connected to a connector 22 of the external defibrillator unit connector 24. The electrode pad 20 is positioned over the patient's thorax. The electrode pad 20 has an adhesive surface for holding the electrode adjacent to the skin of the patient. A transthoracic cardiac stimulation conductor 26 is embedded on the electrode pad 20.

Typically, for transthoracic external defibrillation, the electrode pad 20 is positioned on the patient's thorax and from about 100 to about 400 joules of electrical energy is delivered to the chest area in the region of the heart. By the manner in which the shock is applied, only a portion of this energy is actually delivered to the heart and is available to arrest fibrillation. The ultrasound imaging is performed on the left atrium. In contrast, if after running a TEE test it is determined that the left atrium is clear from blood dots, then the combination of the cardiac stimulation conductor 12 and the electrode pad 20 is used to apply the cardiac stimulation therapy. The cardiac stimulation conductor 12 in the sheath 10 in combination with the electrode pad 20 may be used at opposite surfaces of the ventricular myocardium and, in these instances, the energy required to be delivered is considerably less.

Specifically, the electrode pad 20 is placed on a patient's chest and a jolt of electricity is given to the patient's heart to convert an abnormal heartbeat to a normal one. The current is applied from the cardiac stimulation conductor 12 that is next to the right atrium and the current travels through the chest's wall to the electrode pad 20, and thereby a path is formed from the right atrium into the left ventricle, which is the normal flow of electrical energy in the heart. The current may also travel in the opposite direction from the electrode pad 20 through the chest wall to the cardiac stimulation conductor 12 that is next to the right atrium. Either the electrode pad 20 or the cardiac stimulation conductor 12 may act as the negative (cathode) conductor or the positive (anode) conductor.

Furthermore, in order to assure that an area in the heart receiving the cardiac stimulation is the area being displayed on the monitor by the echo signals from the TEE probe, the sheath 10 covering the "footprint" of the transducer elements in the TEE probe, is electrically conductive and the cardiac stimulation conductor 12 embedded or integrated therein is acoustically transparent. Also, in order to prevent the energy being applied during defibrillation, pacing, or cardioversion from damaging the conductor in the TEE probe or the esophageal probe, the embodiment further provides an insulation type coating with suitable dielectric strength inside the sheath 10 cavity.

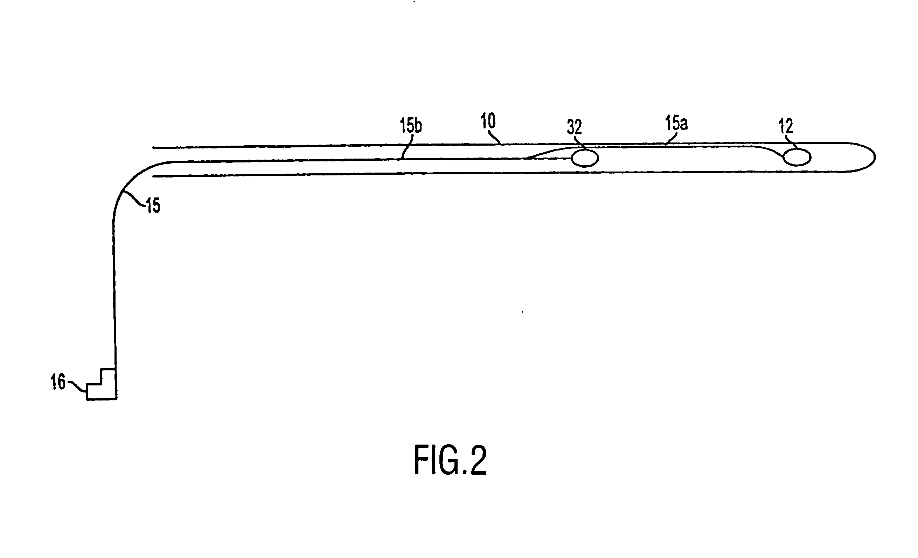

Fig. 2 illustrates a dual conductor sheath, in accordance with an alternative embodiment of the present disclosure. The embodiment of the sheath 10 illustrated in FIG. 1 is modified where, rather than incorporating the single transthoracic cardiac stimulation electrode pad 20, the sheath 10 includes a second cardiac stimulation conductor 32 integrated or embedded on the surface skin of the sheath 10 and it is positioned spaced apart from the first cardiac stimulation conductor 12, which is located at or near the distal end of the sheath 10. The structural and functional characteristics of the sheath 10 are the same as described in Fig. 1 . An electrically conductive, insulated cable 15 includes two conductive wires 15a and 15b and extends from the first cardiac stimulation conductor 12 to the second cardiac stimulation conductor 32 beyond the proximal end of the sheath 10 to the connector 16. The conducting wire 15a is connected to the first cardiac stimulation conductor 12 and the second conducting wire 15b is connected to the second cardiac stimulation conductor 32. The connector 16 then is connected to the defibrillator (not shown).

Thus, the first cardiac stimulation conductor 12 at or near the tip of the sheath 10 is located down at the apex of the heart and the second cardiac stimulation conductor 32 is near to the right atrium when the cardiac stimulation is performed. The current is applied from the second cardiac stimulation conductor 32 to the first cardiac stimulation conductor 12, creating a direct and clean path. As a result, the current does not need to travel through the chest wall, as with the embodiment of FIG. 1, to get to the first cardiac stimulation conductor 12, thereby further reducing the required amount of current the cardiac stimulation treatment. Similar to the conductor in FIG. 1, the first and second conductors 12, 32 may or may not be acoustically transparent.

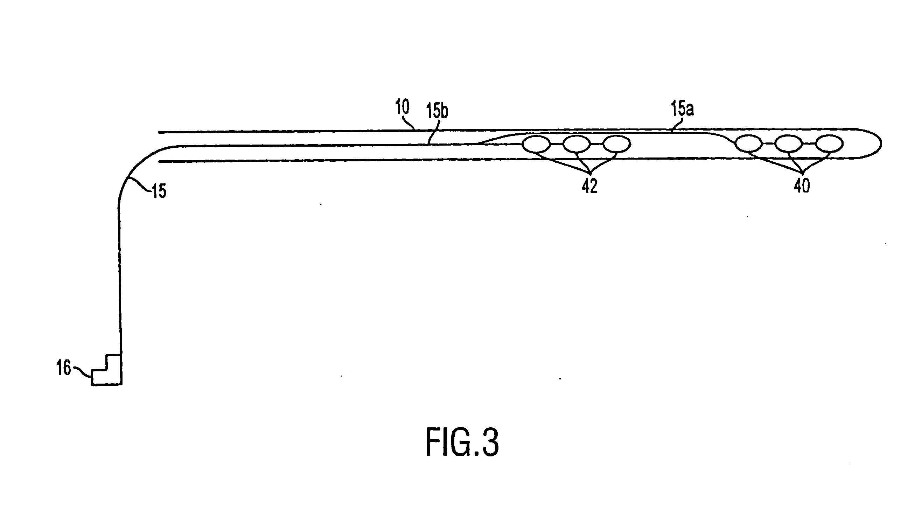

FIG. 3 illustrates a multiple conductor sheath 10, in accordance with an exemplary embodiment of the present invention. The structural and functional characteristics of the sheath 10 are the same as described in FIG. 1. The first and second conductors 12, 32 of FIG. 2 are now two groups of multiple cardiac stimulation conductors 40,42, the first group of conductors 40 positioned near or at the distal end of the sheath 10 and the second group of conductors 42 is positioned spaced apart from the first group of conductors 40 towards the proximal end of the sheath 10. The electrically conductive, insulated cable 15 includes two conductive wires 15a and 15b and extends from the first group of conductors 40 to the second group of conductors 42 beyond the proximal end of the sheath 10 to the connector 16. The conducting wire 15a is connected to the first group of conductors 40 and the second conducting wire 15b is connected to the second group of conductors 42.

The first and second group of conductors 40,42 function as two electrodes for a conduction path to complete the circuit. When current is applied for the cardiac stimulation treatment, the current would flow through the path of least resistance. Thus, rather than "forcing" the current to flow through one path, the current would flow through the path of least resistance, thereby applying with certainty either the defibrillation or the cardioversion therapy. As a result, the amount of energy required for the cardiac stimulation therapy is reduced and there is a high degree of certainty of being successful when applying the cardiac stimulation treatment to the patient. Similar to the conductor in FIG. 1, the first and second groups of conductors 40,42 are acoustically transparent.

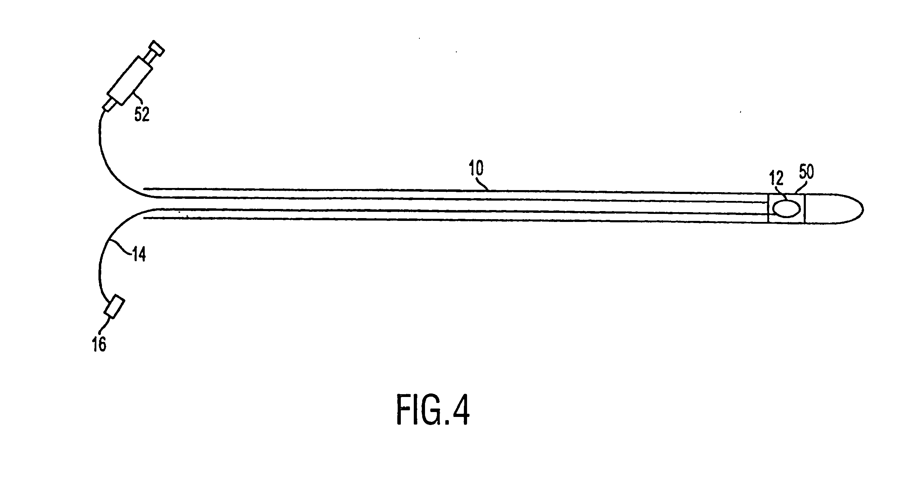

FIG. 4 illustrates a single conductor sheath with inflatable balloon and associated tubing, in accordance with an exemplary embodiment of the present disclosure The structural and functional characteristics of the sheath 10 and the cardiac stimulation conductor 12 are the same as described in FIG. 1. In some occasions, the sheath 10 covering the TEE probe and inserted through the esophagus, may not be in tight contact with the sides of the esophagus. Thus, in order to close the gap between the esophagus and the sheath 10, a balloon 50 would be positioned behind the cardiac stimulation conductor 12 at or near the distal end of the sheath 10. A syringe 52 may be used to inflate the balloon. A user would then inflate the balloon 50 that is positioned behind the cardiac stimulation conductor 12, thereby pushing the cardiac stimulation conductor 12 into the wall of the esophagus. The embodiment illustrated in FIG. 4 may be incorporate the pad of FIG. 1, the second conductor of FIG. 2, or the multiple conductors of FIG. 3, such that each conductor may include an inflatable balloon.

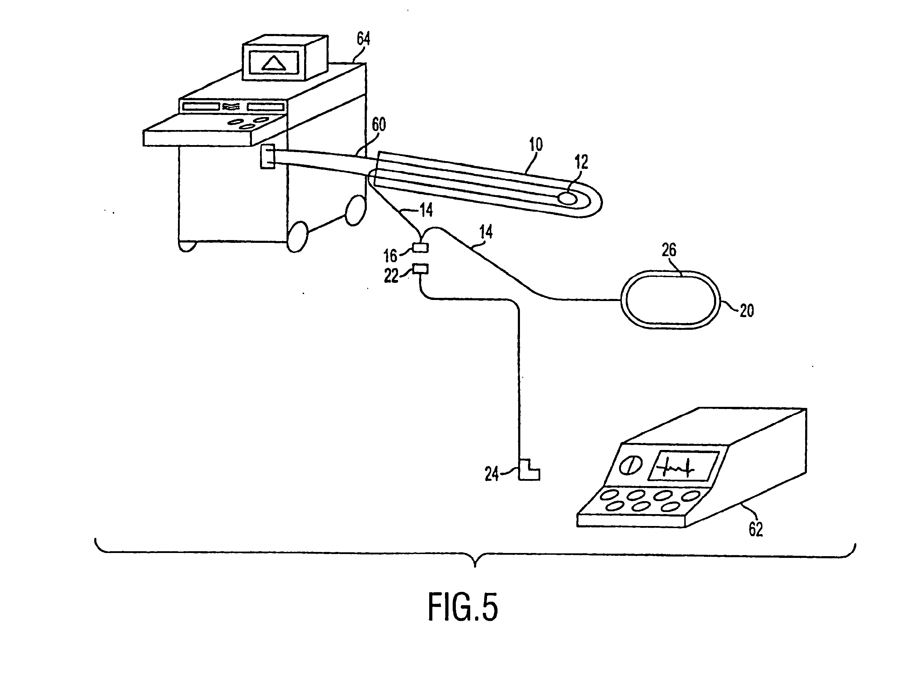

FIG. 5 illustrates a sheath with related equipment, in accordance with an exemplary embodiment of the present disclosure The TEE probe is connected to an ultrasound system 64. The ultrasound system 64 provides the electrical energy to the TEE probe where acoustical waves are created. The TEE probe includes the disposable sheath 10 with the cardiac stimulation conductor 12, which is connected via an electrical conductor to the transthoracic pad. A single connector 16 connects both, the sheath 10 and the electrode pad 20. The connector 16 connects the electrode pad 20 and the sheath 10 covering the TEE probe to a defibrillator 62 via connector set 22,24 to apply defibrillation, pacing, or cardioversion therapy to the patient. In the alternative, the sheath 10 may include a second integrated conductor or multiple integrated conductors substituting the electrode pad 20. The sheath may also be also used to defibrillate a patient in ventricular fibrillation (VF) where the patient cannot be resuscitated with conventional defibrillation techniques.

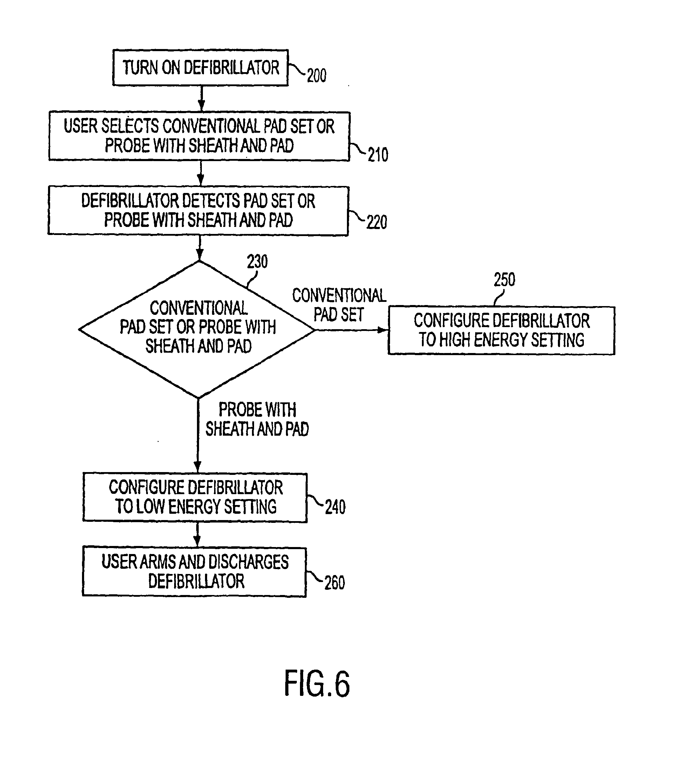

FIG. 6 illustrates a method determining whether a cardiac stimulation system including a defibrillator is using a conventional transthoracic pad set or a combination of the probe including the disposable sheath of the present invention and the single transthoracic pad. At operation 200, the defibrillator is turned on. At operation 210, a user selects the conventional pad set or the combination of the single pad and the probe including the sheath in accordance with the present invention. At operation 220, a defibrillator detects the conventional pad set or the combination of the single pad and the probe including the sheath. At operation 230, the determination is made identifying whether the conventional pad set or the combination of the single pad and the probe including the sheath is connected to the defibrillator. If it is determined that the probe including the sheath and the single pad are used, at operation 240, a processor (not shown) in the defibrillator configures the defibrillator to use a low-energy setting. Accordingly, the patient receives cardiac stimulation using the combination of the single pad and the disposable sheath. However, If it is determined that the conventional pad set is being used, at operation 250, the processor configures the defibrillator to use a high-energy setting. At operation 260, the user arms and discharges the defibrillator.

Accordingly, the sheath of the present invention may be used with a probe or similar device for cardioversion of atrial fibrillation, defibrillation of ventricular fibrillation, or other forms of cardiac stimulation. The sheath of the present invention is an effective device for cardioverting in the esophagus immediately after determining that there are no left atrial blood clots and for reducing the required energy needed to defibrillate and/or cardiovert. Further, the sheath of the present invention eliminates the need for redundant procedures, such as sedation and intubation, eliminates unnecessary anticoagulation therapy, provides for a faster time to cardioversion, and uses less energy minimizing patient risk for myocardial and other tissue damage.

The embodiments of the present disclosure have been shown and described for mere illustration of the invention, which is defined in the claims

## Claims

1. A system providing cardiac stimulation, comprising:
- a first group of conductors (40) functioning as electrodes;
- a second group of conductors (42) functioning as electrodes; and
- a disposable sheath (10) comprising the first group of conductors (40) and the second group of conductors (42) integrated therein,
- wherein the first and the second groups of conductors (40,42) are connectable to a cardiac resuscitation apparatus via a single cable (15) to provide the cardiac stimulation to the patient
**characterized in that**:
- the cable comprises first and second conducting wires (15a, 15b), and the first conducting wire (15a) is connected to the first group of conductors (40) and the second conducting wire (15b) is connected to the second group of conductors (42),
- thereby providing a path of least resistance between one of the conductors in the first group of conductors (40) and one of the conductors in the second group of conductors (42) when the system is in operation.

2. The system as recited in claim 1, wherein the first group of conductors (40) is at or near a distal end of the sheath and the second group of conductors (42) is spaced apart from the first conductor.

3. The system as recited in claim 1, wherein the first and second groups of conductors are acoustically transparent.

4. The system as recited in claim 1, wherein the sheath comprises a flexible membrane material.

5. The system as recited in claim 1, wherein the cardiac stimulation comprises cardioversion, defibrillation, or pacing in atria of the patient.

6. The system as recited in claim 1, wherein the cardiac stimulation comprises cardioversion, defibrillation, or pacing in ventricles of the patient.

7. The system as recited in claim 1, wherein the cardiac stimulation comprises cardioversion, defibrillation, or pacing of any of a plurality of pacemaker sites within a heart of the patient.

8. The system as recited in claim 1, further comprising an inflatable balloon (50) respectively positioned behind each conductor of the second group of conductors to close a gap between the esophagus and the sheath and pushing each conductor against a wall of the esophagus.

9. A system providing cardiac stimulation, comprising:
- a probe insertable through a mouth into an esophagus of a patient;
- a first group of conductors (40) functioning as electrodes;
- a second group of conductors (42) functioning as electrodes; and
- a disposable sheath slidably covering the probe and comprising the first group of conductors (40) and the second group of conductors (42) integrated therein,
- wherein the first and the second groups of conductors (40, 42) are connectable to a cardiac resuscitation apparatus via a single cable (15) to provide the cardiac stimulation to the patient
**characterized in that**:
- the cable comprises first and second conducting wires (15a, 15b), and the first conducting wire (15a) is connected to the first group of conductors (40) and the second conducting wire (15b) is connected to the second group of conductors (42),
- providing a path of least resistance between one of the conductors in the first group of conductors (40) and one of the conductors in the second group of conductors (42) when the system is in operation..

10. The system as recited in claim 9, wherein the sheath comprises an insulation type coating comprising a suitable dielectric strength inside a cavity of the sheath to protect the probe from damage by energy applied during the cardiac stimulation.

## Patentansprüche

1. System zur Verabreichung einer Herzstimulation, das Folgendes umfasst:
- eine erste Gruppe von Leitern (40), die als Elektroden fungieren;
- eine zweite Gruppe von Leitern (42), die als Elektroden fungieren; und
- eine Wegwerfhülle (10) mit darin integrierter erster Gruppe von Leitern (40) und zweiter Gruppe von Leitern (42);
- wobei die erste und die zweite Gruppe von Leitern (40, 42) über ein einzelnes Kabel (15) mit einem Herzwiederbelegungsgerät verbunden werden können, um dem Patienten die Herzstimulation zu verabreichen;
**dadurch gekennzeichnet, dass**:
- das Kabel erste und zweite leitende Drähte (15a, 15b) umfasst und der erste leitende Draht (15a) mit der ersten Gruppe von Leitern (40) verbunden ist und der zweite leitende Draht (15b) mit der zweiten Gruppe von Leitern (42) verbunden ist,
- wodurch ein Pfad des geringsten Widerstands zwischen einem der Leiter in der ersten Gruppe von Leitern (40) und einem der Leiter in der zweiten Gruppe von Leitern (42) geschaffen wird, wenn das System in Betrieb ist.

2. System nach Anspruch 1, wobei die erste Gruppe von Leitern (40) am oder nahe dem distalen Ende der Hülle angeordnet ist und die zweite Gruppe von Leitern (42) in einem Abstand vom ersten Leiter angeordnet ist.

3. System nach Anspruch 1, wobei die erste und die zweite Gruppe von Leitern akustisch transparent sind.

4. System nach Anspruch 1, wobei die Hülle ein flexibles Membranmaterial umfasst.

5. System nach Anspruch 1, wobei die Herzstimulation Kardioversion, Defibrillation oder Schrittstimulation in den Vorhöfen des Patienten umfasst.

6. System nach Anspruch 1, wobei die Herzstimulation Kardioversion, Defibrillation oder Schrittstimulation in den Herzkammern des Patienten umfasst.

7. System nach Anspruch 1, wobei die Herzstimulation Kardioversion, Defibrillation oder Schrittstimulation von einer beliebigen einer Vielzahl von Schrittmacherorten im Herzen des Patienten umfasst.

8. System nach Anspruch 1, weiterhin mit einem aufblasbaren Ballon (50), der jeweils hinter jedem Leiter der zweiten Gruppe von Leitern angeordnet ist, um eine Lücke zwischen der Speiseröhre und der Hülle zu schließen und jeden Leiter gegen die Wand der Speiseröhre zu drücken.

9. System zur Verabreichung einer Herzstimulation, das Folgendes umfasst:
- eine durch den Mund in die Speiseröhre eines Patienten einführbare Sonde;
- eine erste Gruppe von Leitern (40), die als Elektroden fungieren;
- eine zweite Gruppe von Leitern (42), die als Elektroden fungieren, und
- eine auf die Sonde aufschiebbare Wegwerfhülle mit darin integrierter erster Gruppe von Leitern (40) und zweiter Gruppe von Leitern (42),
- wobei die erste und die zweite Gruppe von Leitern (40, 42) über ein einzelnes Kabel (15) mit einem Herzwiederbelegungsgerät verbunden werden können, um dem Patienten die Herzstimulation zu verabreichen;
**dadurch gekennzeichnet, dass**:
- das Kabel erste und zweite leitende Drähte (15a, 15b) umfasst und der erste leitende Draht (15a) mit der ersten Gruppe von Leitern (40) verbunden ist und der zweite leitende Draht (15b) mit der zweiten Gruppe von Leitern (42) verbunden ist,
- wodurch ein Pfad des geringsten Widerstands zwischen einem der Leiter in der ersten Gruppe von Leitern (40) und einem der Leiter in der zweiten Gruppe von Leitern (42) geschaffen wird, wenn das System in Betrieb ist.

10. System nach Anspruch 9, wobei die Hülle eine Isolierbeschichtung mit einer geeigneten Durchschlagfestigkeit in einem Hohlraum der Hülle umfasst, um die Sonde gegen Schäden durch die während der Herzstimulation abgegebene Energie zu schützen.

## Revendications

1. Système procurant une stimulation cardiaque, comprenant :
- un premier groupe de conducteurs (40) fonctionnant comme des électrodes;
- un deuxième groupe de conducteurs (42) fonctionnant comme des électrodes; et
- une gaine jetable (10) comprenant le premier groupe de conducteurs (40) et le deuxième groupe de conducteurs (42) qui y sont intégrés;
- dans lequel les premier et deuxième groupes de conducteurs (40, 42) peuvent être connectés à un appareil de réanimation cardiaque par un câble unique (15) pour procurer la stimulation cardiaque au patient
**caractérisé en ce que**
- le câble comprend des premier et deuxième fils conducteurs (15a, 15b), et le premier fil conducteur (15a) est connecté au premier groupe de conducteurs (40) et le deuxième fil conducteur (15b) est connecté au deuxième groupe de conducteurs (42),
- procurant de ce fait un chemin de moindre résistance entre un des conducteurs dans le premier groupe de conducteurs (40) et un des conducteurs dans le deuxième groupe de conducteurs (42) quand le système est en fonctionnement.

2. Système selon la revendication 1, dans lequel le premier groupe de conducteurs (40) est à ou près d'une extrémité distale de la gaine, et le deuxième groupe de conducteurs (42) est distant du premier conducteur.

3. Système selon la revendication 1, dans lequel les premier et deuxième groupes de conducteurs sont acoustiquement transparents.

4. Système selon la revendication 1, dans lequel la gaine comprend un matériau formant membrane flexible.

5. Système selon la revendication 1, dans lequel la stimulation cardiaque comprend une cardioversion, une défibrillation ou une stimulation dans des oreillettes du patient.

6. Système selon la revendication 1, dans lequel la stimulation cardiaque comprend une cardioversion, une défibrillation ou une stimulation dans des ventricules du patient.

7. Système selon la revendication 1, dans lequel la stimulation cardiaque comprend une cardioversion, une défibrillation ou une stimulation de n'importe lequel d'une pluralité de sites de stimulation cardiaque dans un coeur de patient.

8. Système selon la revendication 1, comprenant en outre un ballon gonflable (50) positionné respectivement derrière chaque conducteur du deuxième groupe de conducteurs pour fermer un espace entre l'oesophage et la gaine et pousser chaque conducteur contre une paroi de l'oesophage.

9. Système procurant une stimulation cardiaque, comprenant :
- une sonde qui peut être insérée par une bouche dans un oesophage d'un patient
- un premier groupe de conducteurs (40) fonctionnant comme des électrodes;
- un deuxième groupe de conducteurs (42) fonctionnant comme des électrodes; et
- une gaine jetable qui couvre la sonde de façon à pouvoir glisser et comprend le premier groupe de conducteurs (40) et le deuxième groupe de conducteurs (42) qui y sont intégrés;
- dans lequel les premier et deuxième groupes de conducteurs (40, 42) peuvent être connectés à un appareil de réanimation cardiaque par un câble unique (15) pour procurer la stimulation cardiaque au patient
**caractérisé en ce que**
- le câble comprend des premier et deuxième fils conducteurs (15a, 15b), et le premier fil conducteur (15a) est connecté au premier groupe de conducteurs (40) et le deuxième fil conducteur (15b) est connecté au deuxième groupe de conducteurs (42),
- procurant un chemin de moindre résistance entre un des conducteurs dans le premier groupe de conducteurs (40) et un des conducteurs dans le deuxième groupe de conducteurs (42) quand le système est en fonctionnement.

10. Système selon la revendication 9, dans lequel la gaine comprend un revêtement de type isolant constituant une résistance diélectrique qui convient à l'intérieur d'une cavité de la gaine pour protéger la sonde d'un endommagement par une énergie appliquée pendant la stimulation cardiaque.
